Europäisches Patentamt

(19) European Patent Office

Office européen des brevets

(11) Publication number: **0 034 949**
**A2**

(12) **EUROPEAN PATENT APPLICATION**

(21) Application number: **81300781.2**

(22) Date of filing: **25.02.81**

(51) Int. Cl.³: **A 61 B 17/32**

(30) Priority: **26.02.80 AU 2541/80**

(43) Date of publication of application:
**02.09.81 Bulletin 81/35**

(84) Designated Contracting States:
**AT BE CH DE FR GB IT LI LU NL SE**

(71) Applicant: **SMITH & NEPHEW (AUSTRALIA)**
**PROPRIETARY LIMITED**
**211 Wellington Road**
**Clayton Victoria 3168(AU)**

(72) Inventor: **Gaskell, John Anthony**
**22 Gymea Avenue**
**Rowville Victoria 3178(AU)**

(74) Representative: **Crawford, Andrew Birkby et al,**
**A.A. THORNTON & CO. Northumberland House 303-306**
**High Holborn**
**London WC1V 7LE(GB)**

(54) Scalpel blade remover and collector.

㊇ A device for removing and storing the blade 7 of a scalpel 1 of the type having a blade retaining reduced section 3 at one end and a handle 2 at the other end. A specific objective is to remove the blade 7 from the scalpel handle 2 with a simple reciprocal in-out action, the device being simple and economical to manufacture, capable of easily and safely removing the blade 7 and storing one or more of said blades 7 without the possibility of any loss thereof, and being disposable at the same point as the blades 7.

The device comprises (1) a container 20 adapted to accommodate at least one blade 7, (2) an aperture 27 in said container shaped to allow the introduction of the blade retaining reduced section 3 of the scalpel 1 into said container 20 such that the blade is in opposed relation to the top 21 of the container 20, (3) biassing means 21a associated with the interior of the container 20 to deflect the front end 9 of the scalpel blade 7 upon insertion of the blade retaining reduced section 3 of the scalpel 1 through the container aperture 27, and (4) a pivotal support 28, 28a within the container 20 adjacent the container aperture 27 for supporting the rear end 8 of said blade 7, said support 28, 28a having a recess 29 adapted to receive the reduced section 3 and to permit the lifting of said rear end 8 of the blade 7 when the front end 9 of the blade 7 is deflected by said biassing means 21a and the reduced section 3 is depressed within said recess 29, the wall of the container adjacent the aperture being shaped to consitute an abutment for the rear end of the blade when the handle is axially withdrawn from said container restraining the blade from outward movement therefrom and allowing retention of the blade in the container.

SECTION X—X
FIG. 4.

"SCALPEL BLADE REMOVER AND COLLECTOR"

## Field of the Invention

This invention relates to scalpels (or surgical knives) of the type comprising a handle having a reduced section at one end thereof with an elongated boss on one side of said reduced section, said boss having a recess partially extending along each side thereof, and a blade having an elongated slot therein with a reduced section, said slot being complemental to said recessed boss, such that the blade is retained on said boss when the reduced section of the blade slot engages the recess on the boss.

## Background of the Invention

The traditional scalpel (surgical knife) has been extensively used in surgery for a number of years and would be the most widely used surgical instrument in the world at present.

The scalpel, due to its extensive use in all parts of the world, consists of a standardised range of handles and blades.

It is generally accepted that the handle section of the scalpel, due to its expense, is cleaned, sterilized and re-used after each surgical procedure, while the blade section is used once only thereby ensuring the sharpness of the blade each time it is used.

At present it is difficult to replace the blade due to the blade being firmly locked onto the handle, the danger involved in attempting to manipulate a fairly sharp, small and contaminated blade using fingers which may be accidentally cut and infected. There is a further problem
in storing and transporting used scalpel blades to a point
of disposal.

The generally used present procedure is to remove
the blade from the handle using forceps or similar device
and placing the used blades in a container for transport
to a disposal station. It is subsequently necessary to
clean and sterilize the contaminated forceps and transport
container.

A device has been proposed in applicant's earlier
Australian Patent Application No. 25594/77 for removing
and storing the blade of a scalpel of the type described
hereinabove comprising a container adapted to accommodate
at least one blade, an aperture in said container shaped
to allow the introduction of the blade retaining reduced
section of the scalpel into said container such that the
blade is in opposed relation to the top of the container
and the separation of the inner end of said blade from said
section, abutment means projecting outwardly from said
aperture adapted to abut against the inner end of the blade
when the handle is depressed and removed from said
container restraining the blade from outward movement
therefrom and allowing retention of the blade in the
container.

The above device has a number of disadvantages
in that (1) the blade is removed partially outside the
container thus potentially causing contamination of the
environment including the user, there being also a
possibility of the blade falling outside the container,
(2) the blades may drop out of the container when the
container is held in a specific position, say with the
opening downwardly directed, and (3) the device necessitates
proper specific positioning and relative alignment of the
scalpel within the container and an intermediate depressing
action on the scalpel handle by the user (i.e. prior to
withdrawal of the scalpel handle from the container).

## Summary of the Invention

Accordingly, it is the principal objective of the present invention to provide a device for removing the blade from the scalpel handle with a simple reciprocal in-out action, said device being simple and economical to manufacture, capable of easily and safely removing the blade and storing one or more of said blades without the possibility of any loss thereof, and being disposable at the same point as the blades.

Accordingly, the present invention provides a device for removing and storing the blade of a scalpel of the type having a blade retaining reduced section at one end and a handle at the other end comprising:-

(1) a container adapted to accommodate at least one blade,

(2) an aperture in said container shaped to allow the introduction of the blade retaining reduced section of the scalpel into said container such that the blade is in opposed relation to the top of the container,

(3) biassing means associated with the interior of the container to deflect the front end of the scalpel blade upon insertion of the blade retaining reduced section of the scalpel through the container aperture, and

(4) a pivotal support within the container adjacent the container aperture for supporting the rear end of said blade, said support having a recess adapted to receive the reduced section and to permit the lifting of said rear end of the blade when the front end of the blade is deflected by said biassing means and the reduced section is depressed within said recess,
the wall of the container adjacent the aperture being shaped to constitute an abutment for the rear end of the blade when the handle is axially withdrawn from said container restraining the blade from outward movement therefrom and allowing retention of the blade in the container.

## Preferred Embodiments of the Invention

Conveniently, the biassing means may consist of a depressed section of the container wall, e.g. the top of the container.

Conveniently, the recessed pivotal support may consist of a pair of opposed, inclined, rounded shoulders upwardly extending from the base of the container aperture.

Advantageously, the abutment wall may include an inwardly turned lip adjacent the container aperture.

## Detailed description of the Invention

The invention will now be described with reference to a preferred embodiment thereof illustrated in the accompanying drawings wherein:

Figure 1 shows a typical scalpel handle and complemental blade before assembly;

Figure 2 shows the assembled scalpel with the blade located on the reduced section of the scalpel handle;

Figure 3 is a front view of the device according to the invention (shown in Figure 6 hereinbelow);

Figure 4 is a view along Section X-X of Figure 3;

Figure 5 is a view along Section Z-Z of Figure 4; and

Figure 6 is an oblique view of the device according to the invention.

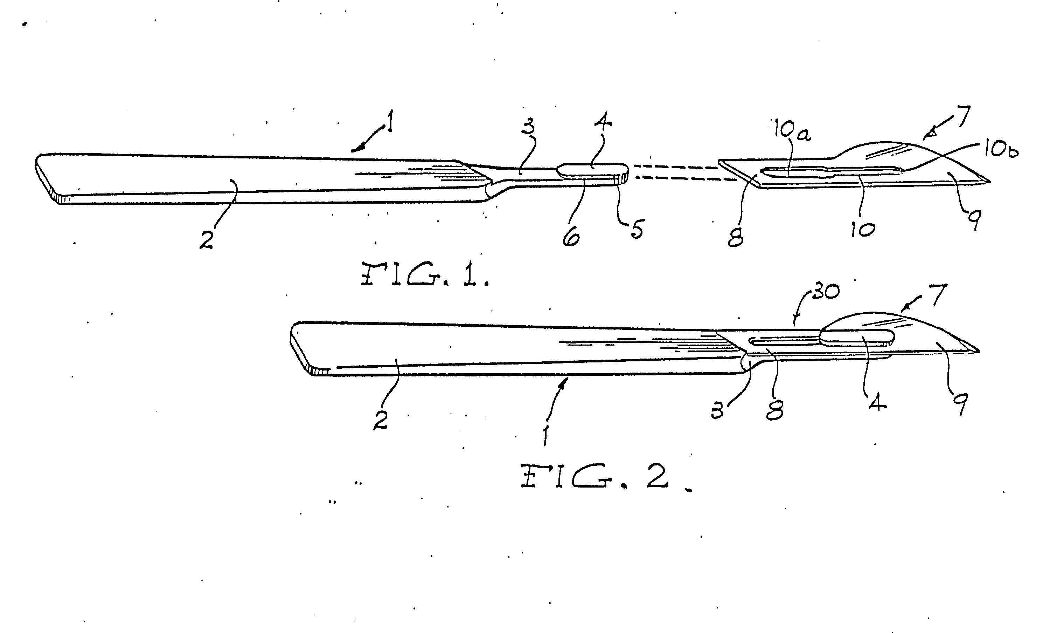

Referring to Figures 1 and 2, a scalpel handle generally indicated as 1 consists of a main section 2 and a reduced section 3 having a raised elongated boss 4 thereon .coextensive with the end 5 of the reduced section as shown. The boss 4 has a recess 6 partially extending along both sides of the boss from its extremity 5.

A scalpel blade, generally indicated as 7, has a rectangular portion 8, a curved portion 9 and an elongated slot 10 having a section 10a and a reduced section 10b. The slot section 10a is adapted for complemental engagement with boss 4 and the reduced section 10b is adapted for location in the recesses 6, such that locked assembly of the scalpel blade 7 and handle occurs when the slot section 10a of the blade is superimposed on boss 4 and the blade is moved towards the handle section 2 (Figure 2), the interengagement of recess 6 and reduced slot 10b locking the blade 7 relative to the blade handle 1.

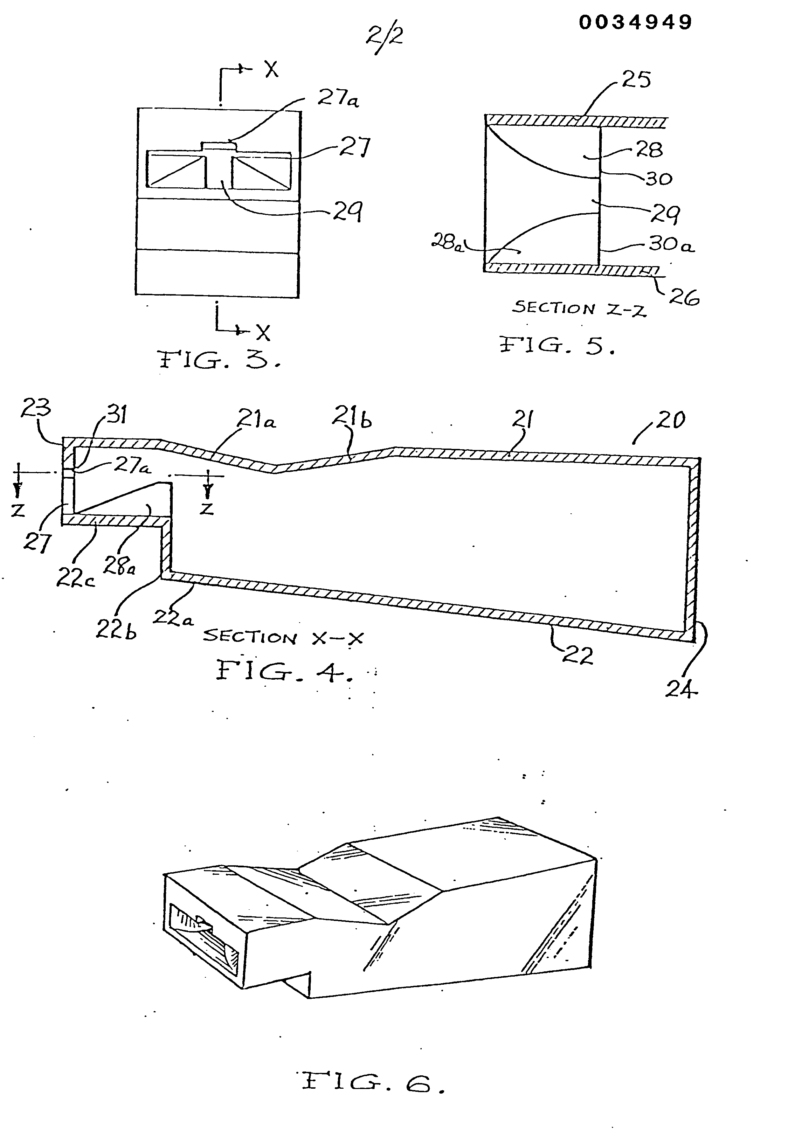

Referring to Figures 3 to 5 of the drawings, a container generally indicated as 20 has top and bottom, and front and rear walls, generally indicated as 21 and 22, and 23 and 24 respectively, as well as side walls 25 and 26.

Bottom wall 22 has a sloping section 22a, an upright section 22b and a substantially horizontal section 22c.

Front wall 23 has a rectangular aperture 27 which includes a narrow section 27a adapted to receive the boss 4 of the reduced section 3 of the scalpel handle 1.

A pair of rounded, inclined shoulders 28, 28a extend from the base of the aperture 27 rearwardly along horizontal section 22c to the end thereof to form a narrow recess 29 therebetween to accommodate the reduced section 3 of the scalpel handle 2. Recess 29 has a depth greater than the thickness of the reduced section 3. The tops of the shoulders 28 and 28a are indicated as 30, 30a and are at a height above the section 22c approximately identical with aperture 27.

The top wall 21 of the container 20 has a depressed section 21a, 21b.

This represents the most efficacious inclination of section 21a which is the section deflecting the scalpel blade 7 upon insertion thereof into the container 10.

In practice, when the contaminated blade supporting reduced section 31 of the scalpel 1 is inserted into the aperture 27, 27a, shoulders 28, 28a guide the blade 7, specifically curved portion 9, until curved portion 9 reaches section 21a of the depressed section 21a, 21b. Reduced section 3 of the scalpel handle 2 is then located within recess 29 and rear end 8 of the blade 7 is supported on the tops 30, 30a of the shoulders 28, 28a. Upon further axial pressure, blade 7 is downwardly deflected by section 21a effecting depression of the reduced section 3 within recess 29 and at the same time lifting of the rear end 8 of the blade 7. When the scalpel handle 2 is withdrawn, the blade 7 abuts against the inner surface of front wall 23 and the blade 7 is then removed from the scalpel handle and drops onto bottom wall 22.

The inner surface of front wall 23 may advantageously be provided with an inwardly turned lip 31 which facilitates the withdrawal of the blade 7 from the scalpel handle 2.

With the construction of the device described above, it will be appreciated that to remove a contaminated blade from a scalpel handle, all that is required is a reciprocal rectilinear movement of the scalpel into and out of the container in contradistinction to the previously proposed device which necessitated an intermediate additional action, i.e. depression or pivotal depression of the scalpel handle prior to withdrawal thereof.

It will furthermore be appreciated that as the abutment for the rear end of the blade is, according to this invention, located within the container (i.e. rear surface of front wall), there is no possibility of contamination of environment or user during the removal of the blade. Furthermore, in view of the depressed section of the container and the disposition of the shoulders, there is virtually no possibility of loss of blades in any position of the device.

CLAIMS:

1.        A device for removing and storing the blade 7 of a scalpel 1 of the type having a blade retaining reduced section 3 at one end and a handle 2 at the other end comprising:

(a) a container 20 adapted to accommodate at least one blade 7,

(b) an aperture 27 in said container shaped to allow the introduction of the blade retaining reduced section 3 of the scalpel 1 into said container 20 such that the blade is in opposed relation to the top 21 of the container 20,

(c) biassing means 21a associated with the interior of the container to deflect the front end 9 of the scalpel blade 7 upon insertion of the blade retaining reduced section 3 of the scalpel 1 through the container aperture 27, and

(d) a pivotal support 28, 28a within the container 20 adjacent the container aperture 27 for supporting the rear end 8 of said blade 7, said support 28, 28a having a recess 29 adapted to receive the reduced section 3 and to permit the lifting of said rear end 8 of the blade 7 when the front end 9 of the blade 7 is deflected by said biassing means 21a and the reduced section 3 is depressed within said recess 29, the wall of the container adjacent the aperture being shaped to constitute an abutment for the rear end of the blade when the handle is axially withdrawn from said container restraining the blade from outward movement therefrom and allowing retention of the blade in the container.

2.        Device as claimed in Claim 1 wherein the biassing means consists of a depressed section 21a of the container wall.

3.      Device as claimed in Claim 2 wherein said depressed section is a section 21a of the top 21 of the container 20.

4.      Device as claimed in any one of the preceding claims wherein the recessed pivotal support consists of a pair of opposed, inclined, rounded shoulders 28, 28a upwardly extending from the base of the container aperture 27.

5.      Device as claimed in any one of the preceding claims wherein the abutment wall 23 includes an inwardly turned lip 31 adjacent the container aperture 27,27a.

FIG. 1.

FIG. 2.

FIG. 3.

FIG. 5.

SECTION Z-Z

SECTION X-X

FIG. 4.

FIG. 6.